# EUROPEAN PATENT APPLICATION

(11) **EP 4 233 716 A2**
(43) Date of publication of application: **30.08.2023**
(21) Application number: 23176526.4
(22) Date of filing: 09.07.2020
(51) Int. Cl.: A61B 5/11

(54) **SYSTEMS AND METHODS FOR DETECTING COGNITIVE DECLINE WITH MOBILE DEVICES**

(30) Priority: 10.07.2019 GR 20190100293; 18.07.2019 US 201962875623 P
(62) Divisional of application: 20754063.4
(71) Applicant: Eli Lilly and Company, Indianapolis, IN 46206-6288 (US)
(72) Inventor: CHEN, Richard, Jia Chuan, Indianapolis, 46206-6288 (US); FOSCHINI, Luca, Indianapolis, 46206-6288 (US); JANKOVIC, Filip, Aleksandar, Indianapolis, 46206-6288 (US); JUNG, Hyun, Joon, Indianapolis, 46206-6288 (US); KOURTIS, Lampros, Indianapolis, 46206-6288 (US); MALJKOVIC, Vera, Indianapolis, 46206-6288 (US); MARINSEK, Nicole, Lee, Indianapolis, 46206-628 (US); PUGH, Melissa, Anna Maria, Indianapolis, 46206-6288 (US); SHEN, Jie, Indianapolis, 46206-6288 (US); SIGNORINI, Alessio, Indianapolis, 46206-6288 (US); SONG, Han, Hee, Indianapolis, 46206-6288 (US); SUNGA, Marc, Orlando, Indianapolis, 46206-6288 (US); TRISTER, Andrew, Daniel, Indianapolis, 46206-6288 (US); TSENG, Belle, Indianapolis, 46206-6288 (US); YAARI, Roy, Indianapolis, 46206-6288 (US)
(74) Representative: J A Kemp LLP

(57) **Abstract**

Embodiments of the present disclosure relate systems and methods for detecting cognitive decline of a subject using passively obtained data from at least one mobile device. In an exemplary embodiment, a computer-implemented method comprises receiving passively obtained data from at least one mobile device. The method further comprises generating digital biomarker data from the passively obtained data. The method further comprises analyzing the digital biomarker data to determine whether the subject is exhibiting signs of cognitive decline.

## Description

### FIELD OF THE DISCLOSURE

The present disclosure relates to systems and methods for detecting cognitive decline with one or more mobile devices. More particularly, the present disclosure relates to systems and methods for detecting cognitive decline using passively obtained sensor measurements collected by one or more mobile devices.

### BACKGROUND OF THE DISCLOSURE

Millions of people worldwide live with cognitive impairment, such as dementia or Alzheimer's disease. Despite the prevalence of people living with cognitive impairment, early diagnosis of cognitive decline is a clinical challenge because early symptoms are subtle and oftentimes attributed to normal aging. As such, there is a need for improved systems and methods for detecting cognitive decline as early as possible.

### SUMMARY

Embodiments of the present disclosure relate to detecting cognitive decline using passively collected sensor measurements from one or more mobile devices. Exemplary embodiments include, but are not limited to, the following examples.

According to one aspect, the present disclosure is directed to a computer-implemented method for detecting cognitive decline of a subject, the method comprising: receiving passively obtained data recorded by at least one mobile device of the subject over an observation period of multiple days, the passively obtained data comprising data regarding at least one of (i) a number of incoming messages received by the mobile device and (ii) a number of outgoing message sent by the mobile device; processing the passively obtained data to generate digital biomarker data; analyzing the digital biomarker data to determine whether the subject is experiencing cognitive decline; and generating a user notification to at least one of the subject and another user regarding the results of the analysis.

According to another aspect, the present disclosure is directed to a computer-implemented method for detecting cognitive decline of a subject, the method comprising: receiving passively obtained data recorded by at least one mobile device of the subject over an observation period of multiple days, the passively obtained data comprising at least one of (i) a time-of-day (ToD) of first-observed subject movement for each day in the observation period, (ii) a ToD of first-observed subject pace for each day in the observation period, (iii) a ToD of last-observed subject movement for each day in the observation period, and (iv) a ToD of last-observed subject pace for each day in the observation period; processing the passively obtained data to generate digital biomarker data; analyzing the digital biomarker data to determine whether the subject is experiencing cognitive decline; and generating a user notification to at least one of the subject and another use regarding the results of the determination.

According to another aspect, the present disclosure is directed to a computer-implemented method for detecting cognitive decline of a subject, the method comprising: receiving passively obtained data recorded by at least one mobile device of the subject over an observation period of multiple days, the passively obtained data comprising data regarding observed stride lengths of the subject; processing the passively obtained data to generate digital biomarker data; analyzing the digital biomarker data to determine whether the subject is experiencing cognitive decline; and generating a user notification to at least one of the subject and another user regarding the results of the analysis.

According to another aspect, the present disclosure is directed to a computer-implemented method for detecting cognitive decline of a subject, the method comprising: receiving passively obtained data recorded by at least one mobile device of the subject over an observation period of multiple days, the passively obtained data comprising data regarding a number of exercise bouts during the observation period; analyzing the passively obtained data to determine whether the subject is experiencing cognitive decline; and generating a user notification to at least one of the subject and another user regarding the results of the analysis.

According to another aspect, the present disclosure is directed to a computer-implemented method for detecting cognitive decline of a subject, the method comprising: receiving passively obtained data recorded by at least one mobile device of the subject over an observation period of multiple days, the passively obtained data comprising data regarding a number of times the subject viewed a mobile clock application for telling time on the at least one mobile device, wherein each time the subject viewed the mobile clock application is associated with a viewing duration; processing the passively obtained data to generate digital biomarker data; analyzing the passively obtained data to determine whether the subject is experiencing cognitive decline; and generating a user notification to at least one of the subject and another user regarding the results of the analysis.

According to another aspect, the present disclosure is directed to a computer-implemented method for detecting cognitive decline of a subject, the method comprising: receiving passively obtained data recorded by at least one mobile device of the subject over an observation period of multiple days, the passively obtained data comprising data characterizing the manner in which the user types while composing outgoing messages sent by the communication device; processing the passively obtained data to generate digital biomarker data; analyzing the digital biomarker data to determine whether the subject is experiencing cognitive decline; and generating a user notification to at least one of the subject and another user regarding the results of the analysis.

According to yet another aspect, the present disclosure is directed to a computer-implemented method for detecting cognitive decline of a subject, the method comprising: receiving passively-obtained time-series data of one or more user activities recorded by at least one mobile device of the subject over an observation period of multiple days; processing the passively obtained time-series data using a frequency analysis to convert the time-series data into a frequency power spectrum; calculating an amount of spectral energy in the frequency power spectrum between a first frequency threshold and a second frequency threshold; generating digital biomarker data based on the calculated amount of spectral energy; analyzing the digital biomarker data to determine whether the subject is experiencing cognitive decline; and generating a user notification to at least one of the subject and another user regarding the results of the analysis.

### BRIEF DESCRIPTION OF THE DRAWINGS

The above-mentioned and other features and advantages of this disclosure, and the manner of attaining them, will become more apparent and will be better understood by reference to the following description of embodiments of the invention taken in conjunction with the accompanying drawings, wherein:
FIG. 1 is a schematic drawing of an illustrative system for detecting cognitive decline using one or more mobile devices, according to at least one embodiment of the present disclosure.
FIG. 2 is a block diagram of illustrative components for detecting cognitive decline using passively collected data from one or more mobile devices, according to at least one embodiment of the present disclosure.
FIG. 3 is a flow diagram of a method for determining cognitive decline using passively collected data from one or more mobile devices, according to at least one embodiment of the present disclosure.
FIG. 4 is a diagram depicting a data structure for recording, processing, and/or displaying passively collected data from one or more mobile devices, according to at least one embodiment of the present disclosure.
FIG. 5 is a diagram depicting twenty exemplary relevant biomarkers that can be used to detect cognitive decline, according to at least one embodiment of the present disclosure.
FIG. 6 is a flow diagram of a method for analyzing passively collected data from one or more mobile devices to determine cognitive decline, according to at least one embodiment of the present disclosure.
FIG. 7 is another flow diagram of a method for analyzing passively collected data from one or more mobile devices to determine cognitive decline, according to at least one embodiment of the present disclosure.
FIG. 8 is a diagram depicting exemplary time-series data and frequency spectrum data that illustrates operation of the method depicted in FIG. 7, according to at least one embodiment of the present disclosure.
FIG. 9 is another flow diagram of a method for analyzing passively collected data from one or more mobile devices to determine cognitive decline, according to at least one embodiment of the present disclosure.
FIG. 10 is a block diagram of illustrative computer system for implementing a system and/or method for detecting cognitive decline using passively collected data from one or more mobile devices, according to at least one embodiment of the present disclosure.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

Common screening tools for cognitive impairment do not consistently detect initial stages of cognitive decline. More sensitive tests that achieve better results require highly specialized and trained rater personnel and lengthy duration of testing, but are also limited by rater bias, cultural bias, educational bias, and practice effects. Also, the limited availability and/or capacity of the current healthcare environment makes widespread screening difficult to achieve.

Computerized efforts have been made to alleviate these limitations. For example, computer-based cognitive assessment tests such as the Cambridge Neuropsychological Test Automated Battery (CANTAB) consist of a battery of neuropsychological tests, administered to subjects using a touch screen computer. However, such neuropsychological tests require a subject to intentionally devote time and attention to complete a test consisting of a series of tasks that evaluate different areas of the subject's cognitive function. As a result, subjects generally do not seek out or complete such tests unless they already suspect that they may be suffering from cognitive decline, which impedes early diagnosis of cognitive decline. Furthermore, such tests generally require that the subject devote significant time and attention to completing the required tasks, at added costs, both direct and indirect, to the healthcare system.

The embodiments disclosed herein provide a solution to these problems that is rooted in computer technology. Specifically, the embodiments disclosed herein use mobile devices that are carried and/or used by subjects during their daily lives to passively collect various parameter data about a subject as they go about their everyday activities. This passively collected parameter data is then analyzed to determine whether a subject may be experiencing cognitive decline. Because mobile devices (e.g., smartphones and/or smartwatches) are ubiquitous and carried by many people throughout the day, this solution provides advantages over the conventional embodiments. For example, the need for a subject to first identify he/she is experiencing cognitive decline may be reduced and/or eliminated. Because parameter data is collected passively while the user conducts his/her usual activities, any intrusion into the user's normal life and routine is decreased. Together, the passively collection of data parameters and relative ubiquity of mobile devices enable very early detection of possible cognitive decline indicative of more serious conditions, such as Alzheimer's disease. Furthermore, the need to actively engage with a specialized rater or computerized screening tool is reduced.

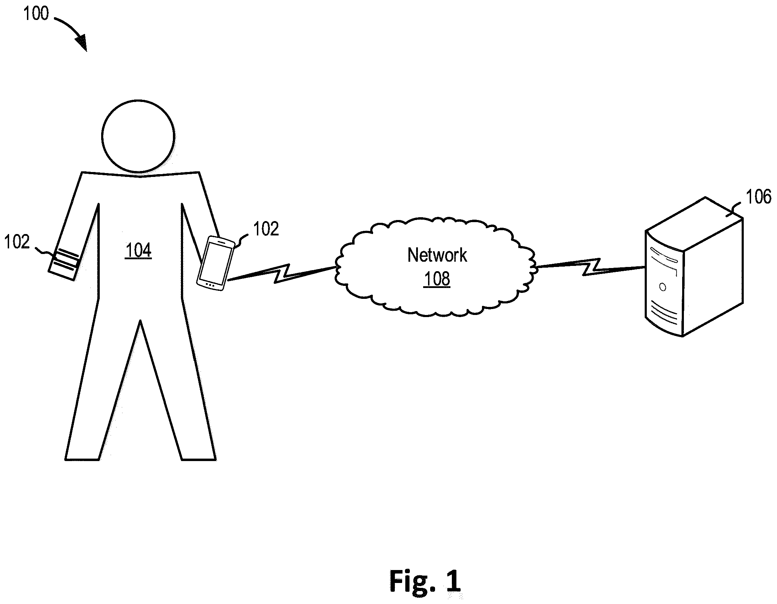

FIG. 1 is a schematic drawing of an illustrative system 100 for detecting cognitive decline using one or more mobile devices 102, according to at least one embodiment of the present disclosure. This drawing is merely an example, which should not unduly limit the scope of the claims. One of ordinary skill in the art would recognize many variations, alternatives, and modifications.

The system 100 includes one or more mobile devices 102 and a subject 104. The mobile device 102 may be any type of electronic device that can be attached to, worn by, carried with, and/or used by the subject 104 to passively sense data about the subject 104 using one or more sensors incorporated into the mobile device 102. Exemplary mobile devices 102 include, but are not limited to, smartphones, smart watches, smart tablets, smart rings, smart suits, pedometers, heart-rate monitors, sleep sensors, and/or the like.

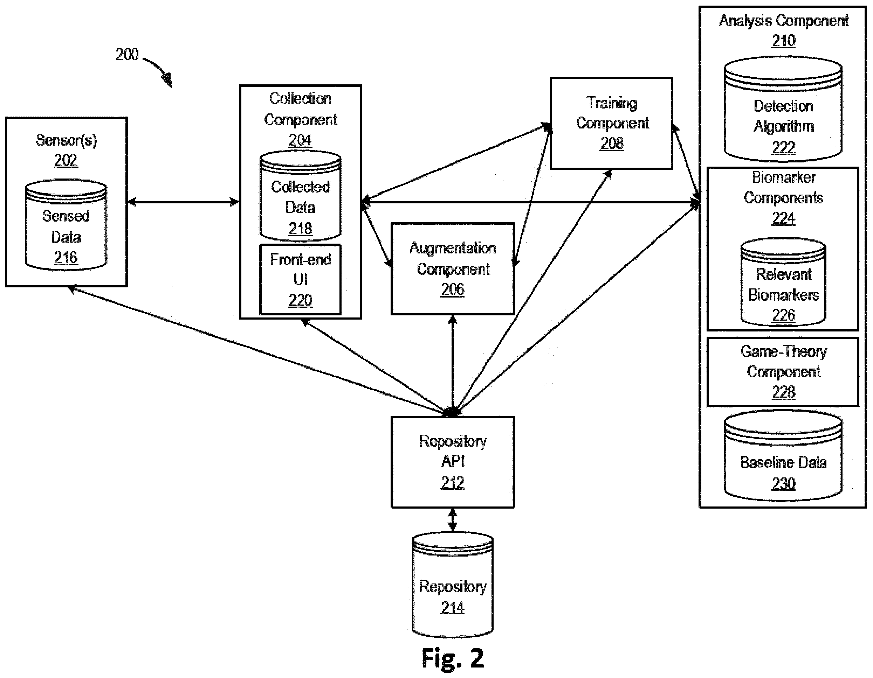

The passively sensed data by the mobile device 102 may correspond to any number of a variety of physiological parameters, behavioral parameters, and/or environmental parameters (collectively referred to herein as "sensed data"). As described in more detail below, the sensed data and/or other data (see FIG. 2) is used to detect cognitive decline. In some embodiments, the mobile device 102 passively collects the sensed data using electrical, mechanical, and/or chemical means during ordinary use of the mobile device 102 by the subject 104 without requiring any additional steps or inputs by the subject 104. In other words, the subject 104 need not alter any aspect of his or her regular daily interaction with the mobile device 102. In some embodiments, the mobile device 102 gathers some of the collected data upon request (e.g., a survey indicative of energy). A single mobile device 102 or multiple mobile devices 102 may collect the collected data.

In some embodiments, the mobile device 102 includes components (e.g., the components 200 depicted in FIG. 2) configured to analyze the sensed data and detect cognitive decline of the subject 104 based on the sensed data. Additionally, or alternatively, the mobile device 102 transmits the sensed data to a server 106 via a network 108 and the server 106 includes components (e.g., the components 200 depicted in FIG. 2) configured to detect cognitive decline of the subject 104 based on the collected data.

The network 108 may be, or include, any number of different types of communication networks such as, for example, a bus network, a short messaging service (SMS), a local area network (LAN), a wireless LAN (WLAN), a wide area network (WAN), the Internet, a P2P network, custom-designed communication or messaging protocols, and/or the like. The network 108 may include a combination of multiple networks.

FIG. 2 is a block diagram of illustrative components 200 for detecting cognitive decline using one or more mobile devices 102, according to at least one embodiment of the present disclosure. This drawing is merely an example, which should not unduly limit the scope of the claims. One of ordinary skill in the art would recognize many variations, alternatives, and modifications. The components 200 may include one or more sensor(s) 202, a collection component 204, an augmentation component 206, a training component 208, an analysis component 210, a Repository Application Programming Interface (API) 212, and/or a repository 214.

As described above, the one or more mobile devices 102 may include one or more sensor(s) used to passively sense data about the subject 104. For example, the sensor(s) 202 may be configured to sense physiological parameters such as one or more signals indicative of a patient's physical activity level and/or activity type (e.g., using an accelerometer), metabolic level and/or other parameters relating to a human body, such as heart rate (e.g., using a photoplethysmogram), temperature (e.g., using a thermometer), blood pressure (e.g., using a sphygmomanometer), blood characteristics (e.g., glucose levels), diet, relative geographic position (e.g., using a Global Positioning System (GPS)), and/or the like. As another example, the sensor(s) 202 may also be able to sense environmental parameters about the external environment (e.g., temperature, air quality, humidity, carbon monoxide level, oxygen level, barometric pressure, light intensity, sound, and/or the like) surrounding the subject 104. As yet another example, the sensor(s) 202 may also be able to sense and/or record behavioral parameters about the subject, such as data summarizing or characterizing the subject's typing, use of mobile applications running on one or more of the mobile devices, messages (e.g., SMS texts, emails, instant chat messages, phone calls, video calls, and the like) sent and/or received by the mobile devices, use of virtual assistants such as Siri, and the like. The physiological parameters, the environmental parameters, and the behavioral parameters may be collectively referred to herein as sensed data 216.

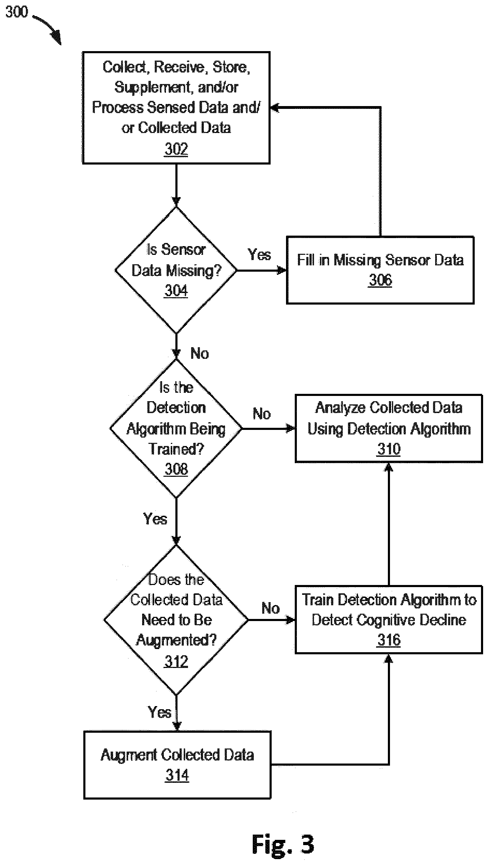

In some embodiments, the collection component 204 is configured to collect, receive, store, supplement, and/or process the sensed data 216 from the sensor(s) 202, as shown in FIG. 3 (block 302). FIG. 3 is a flow diagram of a method 300 for determining cognitive decline using passively collected data from one or more mobile devices, according to at least one embodiment of the present disclosure. This drawing is merely an example, which should not unduly limit the scope of the claims. One of ordinary skill in the art would recognize many variations, alternatives, and modifications. In some embodiments, the collection component 204 receives the sensed data 216 from the sensor(s) 202 and/or collects the sensed data 216 using the sensor(s) (block 302).

As illustrated (block 302), the collection component 204 may additionally or alternatively store the sensed data 216 along with any supplemental data (collectively referred to herein as collected data 218, see FIG. 2). The collected data 218 may be stored in the repository 214 (of FIG. 2). As an example of supplemental data to the sensed data 216, the collection component 204 may collect metadata of the sensed data 216. As a more specific example, the collection component 204 may time stamp the sensed data 216 to determine the beginning of any sensed data 216, the duration of any sensed data 216, occurrences of any sensed data 216, and/or the end of any sensed data 216. Additionally, or alternatively, the collection component 204 may collect psychomotor component data (e.g., tapping speed, tapping regularity, typing speed, sentence complexity, drag path efficiency, reading speed, etc.), and/or metadata about the psychomotor components and/or other interactions of the subject 104 with the mobile device 102 (e.g., word processing, searching, and/or the like). As stated above, the sensed data 216, the psychomotor component data, and/or the metadata may be stored by the collection component 204 as collected data 218 in the repository 214. As described in more detail below, the collected data 218 is used to generate one or more digital biomarkers associated with cognitive decline and analyze the digital biomarkers to detect cognitive decline of the subject 104 (FIG. 1).

In some embodiments, the collection component 204 may determine when and/or how often the sensor(s) 202 senses the sensed data 216, receives the sensed data 216 from the sensor(s) 202, and/or supplements the sensed data 216 to generate the collected data 218. In some embodiments, the collection component 204 performs these tasks without direction from the subject 104. As an example, the collection component 204 instructs the sensor(s) 202 to sample different types of sensed data 216 once per day (e.g., a survey), per hour, per minute (e.g., aggregate physical activity), per second, per 10^{th} of a second, per 100^{th} of a second (e.g., raw accelerometer channels), etc. As another example, the collection component 204 instructs the sensor(s) 202 to sample a first type of sensed data 216 at a constant frequency (e.g., sleep quality data) and a second type of sensed data 216 at a frequency that is adapted to the context of the sensed data. As a specific example, the collection component 204 may instruct the sensor(s) 202 to adapt the sampling frequency for sensed data 216 associated with biomarkers indicative of steps and/or heart rate based on the frequency of the steps and/or heart rate. That is, as steps and/or heart rate increases, the collection component 204 may instruct the sensor(s) 202 to sample the sensed data 216 associated with steps and/or heart rate at a greater frequency, and vice versa.

Referring to FIG. 3, the collection component 204 may query whether any sensor data 216 is missing (block 304). For periods of no data collection due to, for example, a subject 104 not using or wearing a mobile device 102 for any specific period of time, the collection component 204 may fill in missing data (block 306). For example, when an event is triggered, (e.g., when an app is opened or a message is received), the collection component 204 may fill in minutes with no values with zero, which represented the absence of a triggering event in that minute. As another example, the collection component 204 may linearly interpolate gaps of short duration in heart rate (e.g., 1 minute, 5 minutes, 10 minutes, 15 minutes, etc.). As even another example, the collection component 204 may keep all remaining missing data as non-imputed. Missing data (e.g., gaps in behaviors) may be driven due to a person experiencing cognitive decline. As such, the gaps in data may be used to inform whether a person is experiencing cognitive decline. In some embodiments, the collection component 204 groups the collected data 218 into five different channel types: average values, counts, intervals, impulses, and surveys - and computes four general types of features, consisting of aggregates of 1) all minutes, 2) the times of day of different events, 3) daily aggregates, and 4) the durations of continuous "islands" of activity.

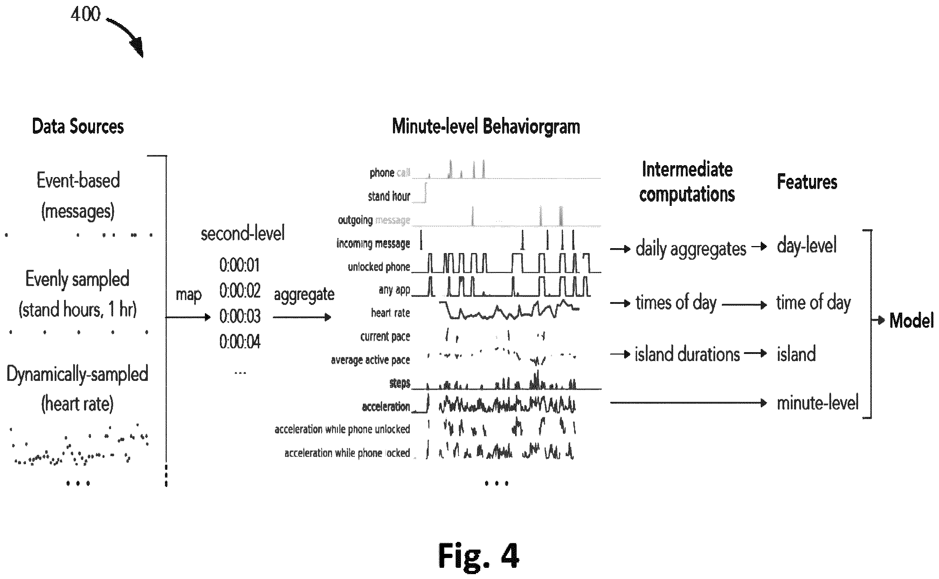

Additionally, or alternatively, the collection component 204 may perform further processing of the sensed data 216 and/or the collected data 218 (block 302). For example, the collection component 204 may map the collected data 218 into a behaviorgram 400, an example of which is illustrated in FIG. 4. The behaviorgram 400 may comprise a data structure that facilitates recording, processing, and/or displaying collected data 218. This data structure may include time-aligned processed data channels with values at a 1-minute resolution, a 1-second resolution, a sub-second resolution, or other time resolutions. To map the collected data 218 into the behaviorgram 400 representation, the collection component 204 may include performing time-alignment between channels, resampling of sources at different time scales, channel-aware aggregations, and handling of missing values. As a specific example, the collection component 204 may align input source timestamps in a timezone-aware fashion and may reassign values from event-based sources to the second in which they occur. The collection component 204 may either sum (for steps, stairs, missed calls, and messages) or average (for pace, stride, heart rate, and survey responses) the values to produce the minute-level-resolution sampling. The collection component 204 may convert input sources representing intervals (e.g., for workout sessions, breathe sessions, stand hours, exercise, phone calls, phone unlocks, and app usage) into minutes by encoding the fraction of the minute covered by the interval. For collected data 218 that requires sub-minute (or sub-second) precision (e.g. fine-motor functions), the collection component 204 may compute statistics at higher time-resolution before aggregating them to a minute-level resolution. For example, the collection component 204 may aggregate accelerometer measurements at 100Hz into minute-level values by averaging the L2 (Euclidean) norm of the X, Y, and Z accelerations taken at each 100th of a second, after applying a low-pass filter or sensor fusion techniques to reduce the effects of gravity.

The behaviorgram 400 may facilitate detecting cognitive decline of a subject 104 by analyzing patterns of associations between different channels. For example, behaviorgram 400 allow inspecting missing data and outliers in one channel within the context of others. As another example, as a data representation format, a behaviorgram 400 makes it easy to capture interactions between different input data sources and may provide a means to conceptually replicate dual-task experiments that are administered in the lab or clinic. More specifically, a subject 104 with cognitive decline may show greater impairment when he/she attempts to do two tasks at the same time (e.g., walking and having a conversation) than when the subject 104 attempts to perform a single task (e.g., only walking). With the behaviorgram 400, it may be easy to add a channel that represents "walking while talking" at the minute level resolution, capturing the average pace during a phone conversation, by merging data channels that represent phone calls and average walking pace.

In some embodiments, the collection component 204 includes a front-end User Interface (UI) component 220 (of FIG. 2) in order for a programmer, clinician, or otherwise, to interact with the collected data 218, the sensor(s) 202, and/or the sensed data 216. While the collection component 204 is depicted as being a separate component than the Repository API 212, the collection component 204 may be incorporated into the Repository API 212.

In some embodiments, both the sensor(s) 202 and the collection component 204 may be implemented on one or more mobile devices, such as devices 102. Components 202 and 204 may comprise both hardware incorporated into or communicably coupled with such mobile devices, as well as software and/or firmware (e.g., a mobile application) configured to implement the functionality described above. In some embodiments, collection component 204 may be implemented on hardware, software, and/or firmware incorporated into or communicably coupled with one or more servers, such as server 106. In some embodiments, collection component 204 may be distributed across both one or more mobile devices (e.g., devices 102) and one or more servers (e.g., servers 106), which work together to implement the functionality described above.

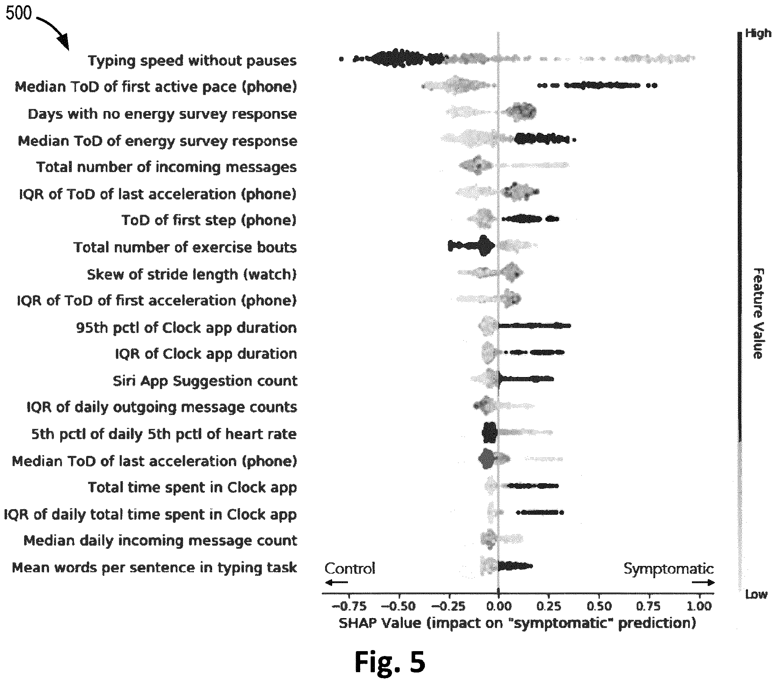

The method 300 may further include querying whether the cognitive decline detection algorithm 222 (of FIG. 2) is being trained (block 308). If the cognitive decline detection algorithm 222 is not being trained, then the method 300 may continue by analyzing the collected data to detect cognitive decline of a subject 104 (block 310). Exemplary embodiments for analyzing collected data to detect cognitive decline are provided in FIGS 5-9.

If, however, the detection algorithm 222 is being trained, then the method 300 may query whether the collected data 218 should be augmented in order to train the detection algorithm 222 (block 312). If the collected data 218 should be augmented, the method 300 may proceed to augmenting the collected data (block 314).

In some embodiments, the augmentation component 206 receives collected data 218 from the collection component 204 to augment the collected data 218. To augment the collected data 218 in order to train the detection algorithm 222, the augmentation component 206 may use features on non-overlapping subsets of the collected data 218. The non-overlapping subset may be, for example, 2-week periods for a total of n (e.g., 3-50) bi-weeks per subject 104: BWi,i ... BWi,n for each subject 104 i. And, the augmentation component 206 may assign each bi-week BW_{i,j} the same label (e.g., healthy control or symptomatic) assigned to subject 104 i. Because, in this instance, the collected data 218 is being used to train the detection algorithm 222 using machine learning techniques (described below), it can be known whether the subject 104 is a healthy control subject 104 (e.g., is not experiencing cognitive decline) or if the subject 104 is experiencing cognitive decline (and if so, the label may optionally further specify what type of cognitive impairment the subject 104 is experiencing, and/or to what degree the subject 104 is experiencing the cognitive impairment). As such, each bi-week BW_{i,j} associated with the subject 104 may be assigned the corresponding cognitive decline or control label of the subject 104. This method may be referred to as Window Slicing in the Time Series Classification. The augmentation component 206 may average BW_{i,j}, into a final score for the subject 104 i. While the augmentation component 206 is depicted as being a separate component than the Repository API 212, the augmentation component 206 may be incorporated into the Repository API 212.

In embodiments, a two-week window may be beneficial because it provides a substantial boost in data size, while at the same time still capturing daily and weekly patterns for a subject 104. In some embodiments, the two-week window may also be beneficial in the event the features computed on the psychomotor tasks were determined for every two weeks. In some embodiments, longer time windows (e.g., three-week, four-week, or month-long windows) may also be used.

Once the collected data 218 has been augmented, the method may include training the detection algorithm 222 to detect cognitive decline (block 316). Alternatively, in the event the collected data 218 does not need to be augmented, the method 300 may proceed to training the detection algorithm 222 (block 316).

In some embodiments, the training component 208 (of FIG. 2) may be used to train the detection algorithm 222. For example, the detection algorithm may be implemented using a convolutional neural network (CNN). For the collected data 218, the training component 208 may use a n-repeat (e.g., 50-500) holdout procedure (where n is the number of subsets of data) to evaluate out-of-sample generalization performance on classifying each bi-week as belonging to a healthy control or symptomatic subject 104. In each of the n iterations, the training component 208 may split the dataset into training and test sets using a 70/30 shuffle split that is stratified by diagnosis (symptomatic vs. healthy control) and grouped by subject 104 (bi-weeks from the same subject 104 all end up in the same set to prevent the model from memorizing a specific subject's 104 pattern). In embodiments, the training component 208 performs hyper-parameter tuning on the training set using grouped 3-fold cross validation. In embodiments, the training component 208 may use Hyperopt to select the following parameters: number of estimators, learning rate, maximum tree depth, and gamma. Hyperopt is described by James Bergstra, Dan Yamins, and David D Cox in "Hyperopt: A python library for optimizing the hyperparameters of machine learning algorithms" at Proceedings of the 12th Python in Science Conference, Citeseer, 13-20, the contents of which are incorporated herein for all purposes. For each combination of parameters, up to m combinations (e.g., 10-50), the training component 208 may evaluate performance of the detection algorithm 222. In embodiments, the training component 208 may select to train on the full training set in the outer split the model hyperparameters that yielded the highest average Area Under the ROC Curve (AUROC) across the three folds. In embodiments, the training component 208 may compute the bi-week model performance metrics on the held-out test set in the outer split. Then, in order to make determinations at the subject-level 104, the training component 208 may aggregate bi-week scores for a subject 104 via soft-voting to rank each subject 104 in the test set. The training component 208 may compute the detection algorithm 222 performance metrics on these scores. Finally, the training component 208 may repeat this procedure for x iterations to estimate average performance metrics and their associated errors.

After the detection algorithm 222 is trained, the method 300 may proceed to analyze collected data 218 recorded over an observation period of multiple days to detect whether a subject 104 is experiencing cognitive decline (block 310). To do so, the analysis component 210 (which may be implemented as part of server 106, as part of the one or more mobile devices 102, or as a combination of the two types of systems) may include a biomarker component 224 that processes the collected data 218 recorded over the observation period to generate digital biomarker data. As used herein, a digital biomarker may refer to a mathematical or statistical function that takes as input at least some of the collected data 218 and outputs a value that may be used by a detection algorithm (e.g., detection algorithm 222) to differentiate between healthy subjects and subjects that may be exhibiting signs of cognitive impairment or decline. A digital biomarker may be used either independently or in combination with other digital biomarkers to detect cognitive decline in a subject. Exemplary digital biomarker data that may be generated from collected data 218 include but are not limited to: biomarkers associated with subject's 104 physical activity, biomarkers associated with the subject's 104 social interactions, biomarkers associated with the subject's 104 word processing, and/or biomarkers associated with the subject's 104 application use.

Method 300 may be modified by adding, deleting, and/or modifying some or all of its steps, according to different embodiments. Whereas method 300 is described as being suitable for both training the detection algorithm 222 and for using the detection algorithm 222, these two tasks may be performed by separate methods in some embodiments. For instance, there may be a first method and/or process for training the detection algorithm 222 using a training set (e.g., created by a large study). Once the detection algorithm 222 is trained, a second method and/or process may be employed to use the detection algorithm 222 to process a new dataset, and output an indication of whether the dataset indicates one or more subjects are experiencing cognitive decline. When the training phase and the classifying phase are split into separate methods, there may be no step for querying whether the detection algorithm is being trained (e.g., step 308, described above).

Some digital biomarkers may be more significant or useful in detecting cognitive decline in a subject 104 than other digital biomarkers. Such biomarkers are referred to herein as relevant biomarkers 226. To determine the relevant biomarkers 226 to generate from collected data 218, the analysis component 210 may include a game-theory component 228. In some embodiments, the game-theory component 228 may use SHapley Additive explanations (SHAP), which combines game theory with local explanations to explain machine learning models (i.e., the detection algorithm 222). In embodiments, the SHAP values are reported for an XGBRegressor model with a pairwise objective function (and default parameters otherwise) that was trained on the collected data 218 for the age-matched cohorts.

Using the aforementioned methods and systems, a set of 20 relevant biomarkers 500 were identified from analysis of data captured from a multi-site 12-week trial conducted by Evidation Health, Inc. on behalf of Eli Lilly and Company and Apple Inc. The study aimed to assess the feasibility of using smart devices to differentiate individuals with mild cognitive impairment (MCI) and early Alzheimer's disease (AD) dementia from healthy controls.

During this 12-week trial, 154 participants provided consent and were screened for eligibility from 12 centers across the United States. Key inclusion criteria were: (1) aged 60-75 years, (2) able to read, write, and speak English, and (3) familiar with digital devices, including having owned and used an iPhone and having an at-home WiFi network.

Participants with MCI had to meet the National Institute on Aging/Alzheimer's Association (NIA-AA) core clinical criteria for MCI due to AD and participants with mild AD dementia had to meet the NIA-AA core clinical criteria for dementia due to AD. For symptomatic participants, a study partner was consented to monitor the compliance with study procedures.

Upon enrollment, each participant was provided an iPhone 7 plus (to be used as their primary phone), an Apple Watch Series 2, a 10.5" iPad pro with a smart keyboard, and a Beddit sleep monitoring device along with apps to collect all sensor and app-usage events during the 12 week study period. In all, 84 healthy controls and 35 symptomatic participants met the inclusion criteria. Participants were asked not to change any therapies for dementia or other medications that could affect the central nervous system over the course of the study, though this was not a requirement for participation.

Over the course of the 12 weeks of data collection, participants were instructed to use their iPhone and Apple Watch as normal, and to keep them charged. Data from sensors in these devices and device usage, including phone lock / unlock, calls, messages, and app history, were passively collected by a study mobile application and transmitted nightly to study servers. Central review of incoming data allowed for outreach when no data were received from devices. Participants with gaps in device data were contacted via email or phone to remind them to use their devices and to troubleshoot any problems.

Participants were also asked to answer two one-question surveys daily (one about mood, one about energy) as well as perform simple activities every two weeks on the Digital Assessment App. The app consisted of several low-burden psychomotor tasks, including a dragging task in which participants dragged one shape onto another, a tapping task in which participants tapped a circle as fast as possible and then as regularly as possible, a reading task in which participants read easy or difficult passages, and a typed narrative task in which participants typed a description of a picture. These activities were selected because they have the potential to be monitored passively in the future. Study procedures included recording and transmitting video and audio of the participants while completing tasks on the Digital Assessment App.

A study platform, similar to the platforms described above in relation to FIGS. 1 and 2, was used to aggregate and analyze the data collected from the iPhone, Apple Watch, and Beddit devices, as well as from the active tests taken on the iPad over the 12-week study period. Data ingested by the platform was time-stamped, checked for consistency, normalized to a standard schema to facilitate data analysis, and saved using an optimized format in a distributed and replicated data store.

Some input sources were sampled at a constant frequency (e.g., sleep quality data), while others were sampled only when relevant events happened (e.g., the time when a specific app was opened). Some input sources were sampled at a frequency that was adapted to the context (e.g., sampling rates of pedometer and heart-rate measurements increased during high-activity and workout periods). Among the evenly-sampled data sources, sampling time ranged from one or more days (e.g., surveys) to one or more minutes (e.g., aggregate physical activity) to sub-second (e.g., raw accelerometer channels sampled at 100 Hz) intervals.

All event streams and time-series raw data sources were mapped into a common representation, similar to the behaviorgram 400 described with reference to FIG. 4. Missing data was handled by filling in with zeros, filled in using linear interpolation, or kept as missing, non-imputed data, as previously described.

The raw data from the study were used to create a set of digital biomarkers to test for efficacy in distinguishing between healthy controls and subjects exhibiting MCI or AD. In total, 996 digital biomarkers were generated from processing of the raw data. These generated digital biomarkers were used to train a convolutional neural network (CNN) to differentiate between a healthy control and a patient suffering from MCI or AD. This training was implemented at least in part using the aforementioned techniques described with reference to, for example, the augmentation component 206 and/or the training component 208 above. Out of the 996 digital biomarkers that were used to train the CNN, the 20 most relevant digital biomarkers are presented as biomarkers 500 in FIG. 5. These 20 digital biomarkers were found to have the greatest impact on the CNN in differentiating between a healthy control and a subject exhibiting MCI or AD. The SHAP values for these top 20 relevant biomarkers 500 that can be used to detect cognitive decline of a subject 104 are illustrated in FIG. 5.

Specifically, the top 20 relevant biomarkers 500 include: typing speed without pauses (i.e., average typing speed in typing task, excluding pauses), median time of day of first active pace sensed by a mobile device 104 during observation period, days with no energy survey response (i.e., fraction of days during observation period without responses to a survey sent out daily to subjects), median time of day of energy survey response (i.e., median time of day of that the daily survey was completed), total number of incoming messages (i.e., sum of incoming messages over all days in the observation period), interquartile range of time of day of last acceleration sensed by the mobile device 102 (i.e., the spread in the times of day that the mobile device 102 is moved for the last time during the observation period), time of day of first step as sensed by the mobile device 102 during the observation period, total number of exercise bouts (i.e., periods spent exercising during observation period), skew of stride length as sensed by mobile device 102 (e.g., a mobile watch), interquartile range of time of day of first acceleration sensed by the mobile device 102 (i.e., the spread in the times of day that the mobile device 102 is moved for the first time during the observation period), 95th percentile of clock application session duration, interquartile range of clock application session duration, smart assistant application (e.g., Siri) suggestion count (i.e., total number of times the smart assistant application was accessed during a specific time period), interquartile range of daily outgoing message counts (i.e., interquartile range of the number of outgoing messages sent per day during observation period), 5th percentile of daily 5th percentiles of heart rate, median time of day of last acceleration sensed by mobile device 102, total time spent in the clock application across all days in observation period, interquartile range of daily total time spent in clock application per day, median daily incoming message count (i.e., median number of incoming messages received per day), mean words per sentence in typing task (i.e., average number of words per sentence in the typing task).

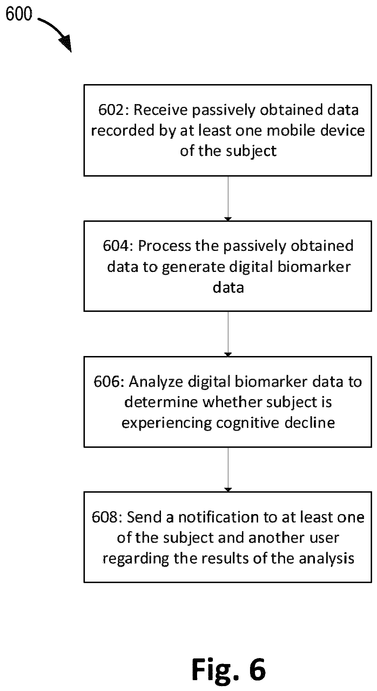

FIG. 6 depicts an exemplary computer-implemented process 600 for using digital biomarkers generated from passively obtained data to detect cognitive decline in a subject, according to some embodiments. Process 600 may be implemented by, for example, collection component 204 and/or analysis component 210, either independently or jointly. Process 600 begins at step 602, which comprises receiving passively obtained data (e.g., sensed data 216 and/or collected data 218) recorded by at least one mobile device of the subject over an observation period of multiple days. The passively obtained data may comprise the raw data recorded by sensors on the at least one mobile device, such as any of the types of raw data mentioned previously.

At step 604, the passively obtained data is processed to generate digital biomarker data. Digital biomarker data may comprise any processed or formatted data that is calculated or derived from, or which summarizes or characterizes, any of the passively obtained data.

For instance, one exemplary category of relevant biomarkers is digital biomarkers generated from passively obtained data regarding at least one of (i) a number of incoming messages received by the mobile device and (ii) a number of outgoing messages sent by the mobile device. Digital biomarkers within this category includes the total number of incoming messages during the observation period, and/or a median number of incoming messages received per day during the observation period. A lower number of total messages and/or messages per day may be associated with lower societal or social engagement, which may be indicative of cognitive decline. Another digital biomarker within this category is a measure of statistical variability in the number of outgoing messages sent by the user's mobile device per day during the observation period. Exemplary measures of statistical variability that may be used include the range, the inter-quartile range, the standard deviation, and/or the variance. Higher statistical variability may be indicative of cognitive decline.

Another exemplary category of relevant biomarkers is digital biomarkers generated from passively obtained data regarding at least one of (i) a time-of-day (ToD) of first-observed subject movement for each day in the observation period, (ii) a ToD of first-observed subject pace for each day in the observation period, (iii) a ToD of last-observed subject movement for each day in the observation period, and (iv) a ToD of last-observed subject pace for each day in the observation period. Digital biomarkers within this category includes a median ToD of first-observed subject pace during the observation period, and/or a median ToD of last-observed subject movement during the observation period. Later median ToDs of first-observed subject paces and/or last-observed subject movement may be indicative of cognitive decline. Another digital biomarker within this category is a measure of statistical variability in the ToD of last-observed subject movement during the observation period, and/or a measure of statistical variability in the ToD of first-observed subject movement during the observation period. Exemplary measures of statistical variability that may be used include the range, the inter-quartile range, the standard deviation, and/or the variance. Higher statistical variability may be indicative of cognitive decline.

Another exemplary category of relevant biomarkers is digital biomarkers generated from passively obtained data regarding observed stride lengths of the subject during the observation period. Digital biomarkers within this category include a statistical skew of the observed stride lengths. A high statistical skew in the observed stride lengths of the subject may be indicative of cognitive decline.

Another exemplary category of relevant biomarkers is digital biomarkers generated from passively obtained data regarding a number of exercise bouts conducted by the subject during the observation period. A low number of exercise bouts may be indicative of cognitive decline.

Another exemplary category of relevant biomarkers is digital biomarkers generated from passively obtained data regarding a number of times the subject viewed a mobile clock application for viewing time on the mobile device(s). Each time the subject viewed the mobile clock application may be associated with a viewing duration. Digital biomarkers within this category include calculating a viewing duration that is greater than or equal to a target percentage of all recorded viewing durations for that respective subject during the observation period. In some embodiments, the target percentage is between 90% and 100%. In some embodiments, the target percentage is between 93% and 97%. In some embodiments, the target percentage is 95%. A high calculated viewing duration may be indicative of cognitive decline. Another example of a digital biomarker within this category is a measure of statistical variability in the viewing durations associated with each time the subject viewed the mobile clock application during the observation period. Higher statistical variability may be indicative of cognitive decline. Another example of a digital biomarker within this category is a total viewing duration over the observation period - a higher total viewing duration may be indicative of cognitive decline. Yet another example of a digital biomarker within this category is a measure of statistical variability in the total daily viewing duration over each day in the observation period, wherein each total daily viewing duration is equal to the sum of all viewing durations during a particular day. Again, higher statistical variability may be indicative of cognitive decline. As before, exemplary measures of statistical variability that may be used include the range, the inter-quartile range, the standard deviation, and/or the variance.

Another exemplary category of relevant biomarkers is digital biomarkers generated from passively obtained data characterizing the manner in which the user types while inputting data into, or interacting with, the mobile device. For instance, the data may characterize the manner in which the user types while composing outgoing messages sent by the communication device. Digital biomarkers within this category include a typing speed excluding pauses, and/or a mean number of words per sentence. A slower typing speed and/or a lower mean number of words per sentence may be indicative of cognitive decline.

At step 606, the digital biomarker data may be analyzed to determine whether the subject is cognitively impaired. As described herein, this analysis may be implemented using a CNN that has been trained to differentiate between a healthy subject and a subject exhibiting MCI and/or AD.

At step 608, a notification may be sent to at least one of the subject and another user regarding the results of the analysis. This notification may comprise any notification or summary based on the results of the analysis. For example, the notification may comprise a summary of the analysis, a probability of cognitive decline, a binary indication of whether cognitive decline was detected, a brain or neuropsychiatric score, a notification to seek treatment or further diagnosis, and the like.

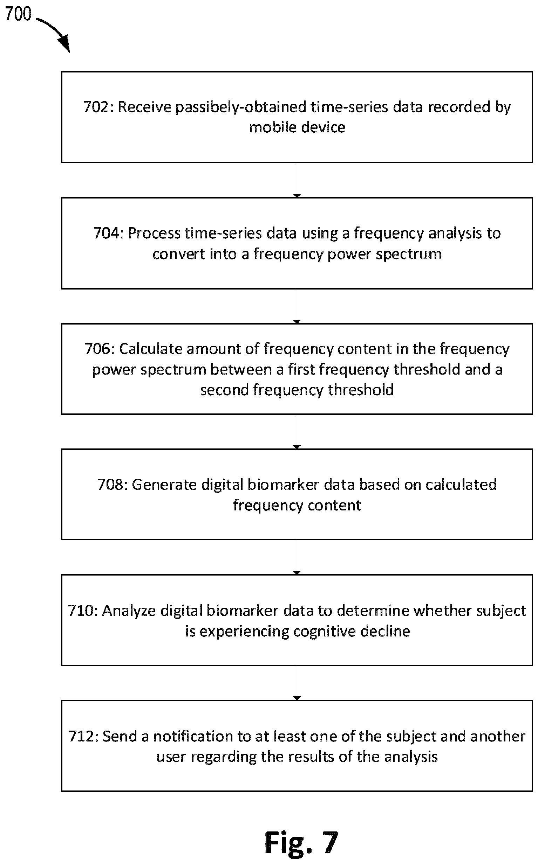

FIG. 7 depicts another exemplary process 700 to detect cognitive decline in a subject, according to some embodiments. Process 700 may also be implemented by, for example, collection component 204 and/or analysis component 210, either independently or jointly. Process 700 begins at step 702, which comprises receiving passively-obtained time-series data of one or more user activities recorded by at least one mobile device of the subject over an observation period of multiple days. Any data having a time-stamp and which was recorded by any of the aforementioned mobile devices may be used. Examples of such time-series data include, but are not limited to, phone calls, outgoing messages, incoming messages, mobile device unlocks, interaction with a mobile application, heart-rate, standing motions, steps, movement, movement while mobile device is unlocked, movement while mobile device is locked, and the like.

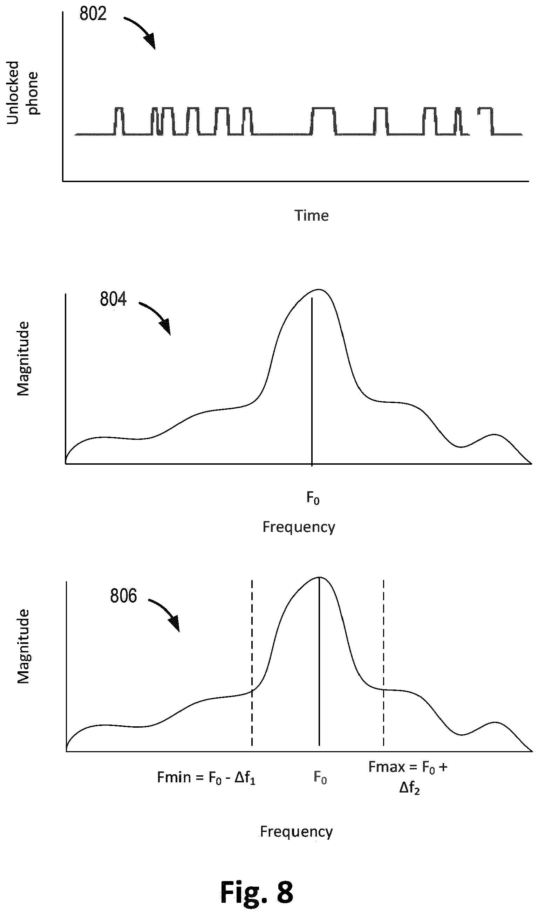

Purely for the sake of illustration, graph 802 in FIG. 8 depicts one exemplary set of time-series data that shows the times at which the subject's mobile device is locked or unlocked. The horizontal axis of graph 802 depicts the passage of time in suitable units, such as seconds, minutes, and/or hours. The vertical axis of graph 802 indicates whether the subject's phone was locked or unlocked - for example, high (a binary 1) may signify the device is unlocked, while low (a binary 0) may signify the device is locked. The time-series data preferably spans data that has been recorded continuously, or substantially continuously, over a period of multiple days (e.g., one week, two weeks, and/or one month).

At step 704, the obtained time-series data is processed using a frequency analysis to convert the time-series data into a frequency power spectrum. Any known frequency analysis that converts time-series data into a frequency power spectrum may be used, such as, but not included to, a Fourier Transform, a Fast Fourier Transform (FFT), a Discrete Fourier Transform (DFT), a wavelet transform, and/or a Lomb-Scargle Periodogram.

An exemplary output of step 704 is depicted in graph 804 in FIG. 8. Graph 804 depicts the frequency power spectrum of the time-series data depicted in graph 802. The horizontal axis of graph 804 depicts frequency, in suitable units such as hertz. The vertical axis of graph 804 depicts the magnitude of the frequency content in the time-series data at that frequency. Since most subject's activities are expected to vary regularly with a regular 24 hour daily cycle, the graph 804 for most subjects will generally have the highest frequency content at or around a frequency F₀ that corresponds to a period of 24 hours, *i.e.,* 1/(24 hours), or 1.157^{∗}10⁻⁵ Hz.

At step 706, process 700 may calculate an amount of frequency content in the frequency power spectrum between a first frequency threshold (Fmin) and a second frequency threshold (Fmax). The frequency thresholds Fmin and Fmax satisfy the inequality Fmin < F₀ < Fmax. Specifically, as depicted in graph 806 in FIG. 8, Fmin may be equal to F₀ - Δf₁, while Fmax may be equal to F₀ + Δf₂. In some embodiments, Δf₁ may equal Δf₂, while in other embodiments, they may not be equal.

Fmin and Fmax define a relatively narrow range of frequencies around F₀, which corresponds to a period of 24 hours. For example, Fmin may be set greater than or equal to the frequency that correspond to a period that is one half hour longer than 24 hours, *i.e.,* 1/(24 hours and 30 minutes), or 1.134^{∗}10⁻⁵ Hz. Or, Fmin may be set greater than or equal to the frequency that corresponds to a period that is one hour longer than 24 hours, *i.e.,* 1/(25 hours), or 1.111^{∗}10⁻⁵ Hz. Similarly, Fmax may be set less than or equal to the frequency that corresponds to a period that is one half hour shorter than 24 hours, *i.e.,* 1/(23 hours and 30 minutes), or 1.182^{∗}10⁻⁵ Hz. Or, Fmax may be set less than or equal to the frequency that corresponds to a period that is one hour shorter than 24 hours, *i.e.,* 1/(23 hours), or 1.208^{∗}10⁻⁵ Hz.

The amount of spectral energy between Fmin and Fmax may be calculated based on the area under the frequency spectrum curve between Fmin and Fmax. In some embodiments, the amount of spectral energy may also be calculated based on the square of the aforementioned area.

At step 708, process 700 generates digital biomarker data based on the calculated amount of spectral energy. In some embodiments, this step may comprise simply using the calculated amount of spectral energy as a digital biomarker. In other embodiments, process 700 may calculate, at step 708, the ratio of (i) the area under the frequency spectrum curve between Fmin and Fmax and (ii) the area under the frequency spectrum curve at all other frequencies that are less than Fmin and greater than Fmax. This ratio may then be used as a digital biomarker.

At step 710, process 700 analyzes the digital biomarker data to determine whether the subject is experiencing cognitive decline. Since healthy subjects exhibit relatively greater regularity and adherence to a 24 hour rhythm in their activities, a relatively high amount of spectral energy between Fmin and Fmax, and/or a relatively high result when computing the ratio described in the previous paragraph, could indicate the subject is not exhibiting signs of cognitive decline. Conversely, subjects exhibiting signs of cognitive decline may exhibit greater irregularity in their activities, and the time-series data recorded from their mobile device(s) may not adhere to a regular 24 hour rhythm. As a result, a relatively low amount of spectral energy between Fmin and Fmax, and/or a relatively small result when computing the ratio described in the previous paragraph, could indicate the subject is exhibiting signs of cognitive decline.

At step 712, a notification may be sent to at least one of the subject and another user regarding the results of the analysis. As before, this notification may comprise any notification or summary based on the results of the analysis. For example, the notification may comprise a summary of the analysis, a probability of cognitive decline, a binary indication of whether cognitive decline was detected, a brain or neuropsychiatric score, a notification to seek treatment or further diagnosis, and/or the like.

The analysis component 210 may use any one or more of the aforementioned digital biomarkers to detect if a subject 104 is experiencing cognitive decline. In some embodiments, the analysis component 210 may categorize the type of cognitive impairment a subject 104 is experiencing based on said digital biomarkers. Combining some or all of the aforementioned multiple digital biomarkers may improve the precision and accuracy of a detection algorithm for detecting cognitive decline.

For example, some or all of the aforementioned digital biomarkers may be used together to train detection algorithm 222 (of FIG. 2). Detection algorithm may take the form of a convolutional neural network (CNN) having one or more layers of nodes, wherein each layer has one or more nodes. During the training phase, the CNN may be trained using training data comprising both the aforementioned digital biomarkers for a population of training subjects, as well as a ground truth label indicating whether each subject for which the digital biomarker was generated was a healthy control, or a subject exhibiting signs of MCI and/or AD. A machine-learning algorithm may be applied to determine a set of weights for some or all of the connections between digital biomarkers and nodes in the first layer of nodes, and also for some or all of the connections between nodes. The weights may be determined such that when they are applied to digital biomarkers generated for subjects where it is not known whether the subjects are healthy or experiencing cognitive decline, the CNN may be used to determine the condition of the subjects. Stated another way, the weights in the CNN may be determined during training of detection algorithm 222 such that when the analysis component 210 applies the weights to digital biomarkers generated from collected data 218 for a subject 104 that is healthy, the analysis component will determine, with a degree of confidence (e.g., percentage likelihood), the subject 104 is healthy. Conversely, when the analysis component 210 applies the weights to digital biomarkers generated from collected data 218 for a subject 104 that is experiencing cognitive decline, the analysis component 210 will determine, with a degree of confidence (e.g., percentage likelihood), the subject 104 is experiencing cognitive decline. Such a detection algorithm 222 employing a CNN may be trained and/or used to detect cognitive decline using any or all of the previously mentioned digital biomarkers.

In some embodiments, the detection algorithm 222 may have been trained on collected data 218 for subjects 104 having different categorizations of cognitive decline. In these embodiments, the analysis component 210 may determine a specific categorization of cognitive decline for a subject 104. For example, the detection algorithm 222 may have been trained on collected data 218 for subjects 104 having mild cognitive impairment and early Alzheimer's disease. As such, the analysis component 210 may determine, by applying the weights determined during the training of the detection algorithm 222, not only whether a subject 104 is healthy or is experiencing cognitive decline, but also if the subject 104 is experiencing cognitive decline, what categorization of cognitive impairment the subject 104 is experiencing, i.e., mild cognitive impairment and early Alzheimer's disease (block 606).

In some embodiments, the detection algorithm 222 may comprise a decision tree that uses digital biomarkers calculated from the raw collected data 218 to determine whether a subject 104 is experiencing cognitive decline. The decision tree may comprise one or more processing steps for calculating digital biomarkers from the raw collected data 218, and/or to compare the processed digital biomarkers against thresholds or expected ranges. Such steps, thresholds, and/or ranges may be derived using the machine learning techniques described herein.

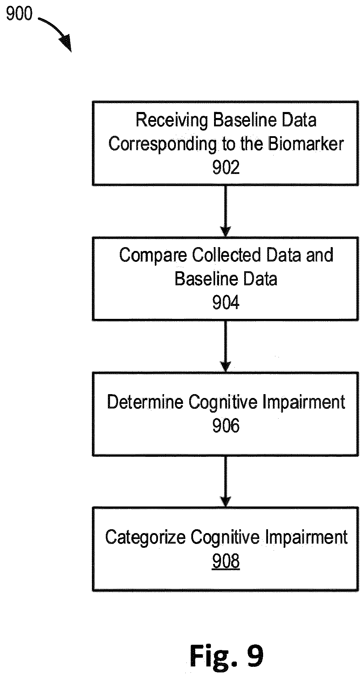

FIG. 9 is another flow diagram of a method 900 for analyzing passively collected data from a mobile device to determine cognitive decline, according to at least one embodiment of the present disclosure. This drawing is merely an example, which should not unduly limit the scope of the claims. One of ordinary skill in the art would recognize many variations, alternatives, and modifications.

At step 902, baseline data corresponding to one or more (or all) of the aforementioned digital biomarkers may be received. Baseline data 230 for a biomarker may be determined during the training of the detection algorithm 222 and may correspond to different baselines when the subject 104 is healthy and/or when the subject is experiencing cognitive decline. More specifically, for each biomarker, baseline data 230 for that biomarker may be determined that indicates when the subject 104 is healthy and when the subject is experiencing cognitive decline. This baseline data may be generated from subjects from the same or similar population as the subject 104 being evaluated, from subjects having the same or similar demographic and/or medical characteristics as the subject 104 being evaluated. In some embodiments, this baseline data may be generated from past measurements obtained from the subject 104 being evaluated. In other words, the baseline data received may be a longitudinal baseline data set that, in some cases, may be unique to each individual subject 104 being evaluated. Then, the baseline data 230 may be compared to digital biomarkers generated from the collected data 218 (block 904) to determine whether the subject 104 is experiencing cognitive decline (block 906) and/or a categorization of cognitive decline (block 908). For example, if the collected data 218 for the biomarker is within a certain percentage (e.g., 0-20%) of baseline data 230 where the baseline data 230 is associated with a subject 104 that is experiencing cognitive decline and/or a subject 104 that is experiencing a specific categorization of cognitive decline, then it may be determined the subject 104 associated with the collected data 218 is experiencing cognitive decline and/or is experiencing a specific categorization of cognitive decline, respectively. As another example, if the collected data 218 for the biomarker is outside of a certain percentage (e.g., 0-20%) of baseline data 230 where the baseline data 230 is associated with a subject 104 is experiencing cognitive decline, then it may be determined the subject 104 associated with the collected data 218 is healthy. As even another example, if the collected data 218 for the biomarker is within a certain percentage (e.g., 0-20%) of baseline data 230 where the baseline data 230 is associated with a subject 104 that is healthy, then it may be determined the subject 104 associated with the collected data 218 is healthy. As another example, if the collected data 218 for the biomarker is outside of a certain percentage (e.g., 0-20%) of baseline data 230 where the baseline data 230 is associated with a subject 104 that is healthy, then it may be determined the subject 104 associated with the collected data 218 is experiencing cognitive decline. As yet another example, if the collected data 118 for the biomarker for a specific subject 104 exhibits a trend towards higher or lower cognitive functioning over time, then it may be determined that the subject 104 is or is not experiencing cognitive decline. The determination of cognitive decline (block 712) and/or the categorization of cognitive decline (block 714) may be communicated to the subject 104 (FIG. 1) or another authorized party, such as a family member and/or a health care provider, to arrange further evaluation and/or treatment.

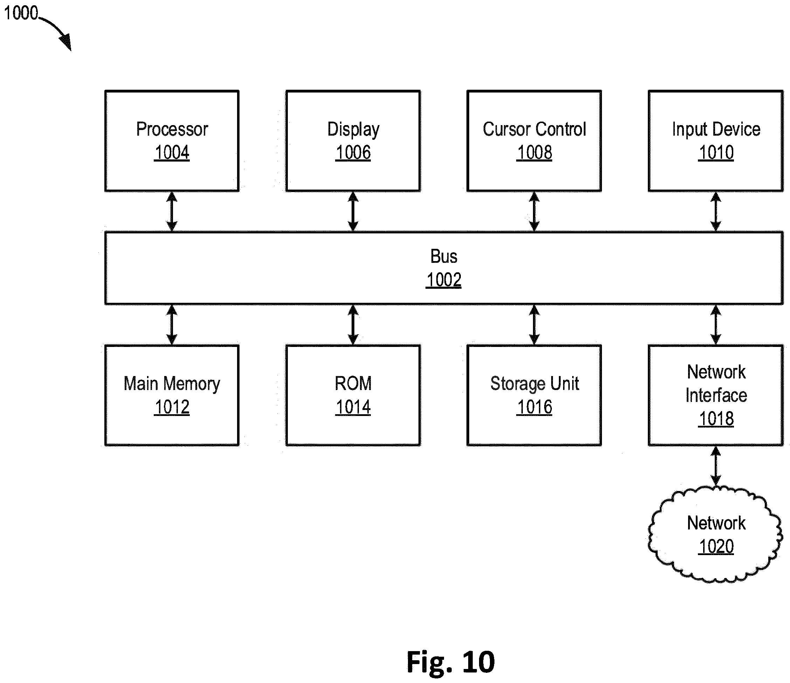

FIG. 10 is a block diagram of illustrative components of a computer system 1000 for implementing a system and/or method for detecting cognitive decline using a mobile device, according to at least one embodiment of the present disclosure. For example, some or all of the functions of the components 200 and/or processes (e.g., steps) of the methods 300, 600, 700, and/or 900 are performed by the computing system 1000. This diagram is merely an example, which should not unduly limit the scope of the claims. One of ordinary skill in the art would recognize many variations, alternatives, and modifications.

The computing system 1000 includes a bus 1002 or other communication mechanism for communicating information between, a processor 1004, a display 1006, a cursor control component 1008, an input device 1010, a main memory 1012, a read only memory (ROM) 1014, a storage unit 1016, and/or a network interface 1088. In some examples, the bus 1002 is coupled to the processor 1004, the display 1006, the cursor control component 1008, the input device 1010, the main memory 1012, the read only memory (ROM) 1014, the storage unit 1016, and/or the network interface 1018. And, in certain examples, the network interface 1018 is coupled to a network 1020 (e.g., the network 108).

In some examples, the processor 1004 includes one or more general purpose microprocessors. In some examples, the main memory 1012 (e.g., random access memory (RAM), cache and/or other dynamic storage devices) is configured to store information and instructions to be executed by the processor 1004. In certain examples, the main memory 1012 is configured to store temporary variables or other intermediate information during execution of instructions to be executed by processor 1004. For example, the instructions, when stored in the storage unit 816 accessible to processor 1004, render the computing system 1000 into a special-purpose machine that is customized to perform the operations specified in the instructions (e.g., the method 300, the method 600, the method 700 and/or the method 900). In some examples, the ROM 1014 is configured to store static information and instructions for the processor 1004. In certain examples, the storage unit 1016 (e.g., a magnetic disk, optical disk, or flash drive) is configured to store information and instructions.

In some embodiments, the display 1006 (e.g., a cathode ray tube (CRT), an LCD display, or a touch screen) is configured to display information to a user of the computing system 1000. In some examples, the input device 1010 (e.g., alphanumeric, and other keys) is configured to communicate information and commands to the processor 1004. For example, the cursor control 1008 (e.g., a mouse, a trackball, or cursor direction keys) is configured to communicate additional information and commands (e.g., to control cursor movements on the display 1006) to the processor 1004.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

Various aspects are described in this disclosure, which include, but are not limited to, the following aspects:
1. A computer-implemented method for detecting cognitive decline of a subject, the method comprising: receiving passively obtained data recorded by at least one mobile device of the subject over an observation period of multiple days, the passively obtained data comprising data regarding at least one of (i) a number of incoming messages received by the mobile device and (ii) a number of outgoing message sent by the mobile device; processing the passively obtained data to generate digital biomarker data; analyzing the digital biomarker data to determine whether the subject is experiencing cognitive decline; and generating a user notification to at least one of the subject and another user regarding the results of the analysis.
2. The computer-implemented method of aspect 1, wherein each message is at least one of a SMS text message, an email, a chat message, a voice call, and a video conference call.
3. The computer-implemented method of any of aspects 1-2, wherein processing the passively obtained data comprises summing all incoming messages received over each day of the observation period to generate a total number of incoming messages, and wherein the digital biomarker data comprises the total number of incoming messages.
4. The computer-implemented method of any of aspects 1-3, wherein processing the passively obtained data comprises calculating a statistical measure of variability in the number of outgoing messages sent by the mobile device over each day in the observation period, and wherein the digital biomarker data comprises the calculated statistical measure of variability in the number of outgoing messages.
5. The computer-implemented method of aspect 4, wherein the calculated statistical measure is an inter-quartile range.
6. The computer-implemented method of any of aspects 1-5, wherein processing the passively obtained data comprises calculating a median number of incoming messages received per day during the observation period, and wherein the digital biomarker data comprises the calculated median number of incoming messages.
7. A computer-implemented method for detecting cognitive decline of a subject, the method comprising: receiving passively obtained data recorded by at least one mobile device of the subject over an observation period of multiple days, the passively obtained data comprising at least one of (i) a time-of-day (ToD) of first-observed subject movement for each day in the observation period, (ii) a ToD of first-observed subject pace for each day in the observation period, (iii) a ToD of last-observed subject movement for each day in the observation period, and (iv) a ToD of last-observed subject pace for each day in the observation period; processing the passively obtained data to generate digital biomarker data; analyzing the digital biomarker data to determine whether the subject is experiencing cognitive decline; and generating a user notification to at least one of the subject and another use regarding the results of the determination.
8. The computer-implemented method of aspect 7, wherein processing the passively obtained data comprises calculating a median ToD of first-observed subject pace during the observation period, and wherein the digital biomarker data comprises the calculated median ToD of first-observed subject pace.
9. The computer-implemented method of any of aspects 7-8, wherein processing the passively obtained data comprises calculating a measure of statistical variability in the ToD of last-observed subject movement during the observation period, and wherein the digital biomarker data comprises the calculated measure of statistical variability in the ToD of last-observed subject movement.
10. The computer-implemented method of aspect 9, wherein the measure of statistical variability is an inter-quartile range.
11. The computer-implemented method of any of aspects 7-10, wherein processing the passively obtained data comprises calculating a measure of statistical variability in the ToD of first-observed subject movement during the observation period, and wherein the digital biomarker data comprises the calculated measure of statistical variability in the ToD of first-observed subject movement.
12. The computer-implemented method of aspect 11, wherein the measure of statistical variability is an inter-quartile range.
13. The computer-implemented method of any of aspects 7-11, wherein processing the passively obtained data comprises calculating a median ToD of last-observed subject movement during the observation period, and wherein the digital biomarker data comprises the calculated median ToD of last-observed subject movement.
14. A computer-implemented method for detecting cognitive decline of a subject, the method comprising: receiving passively obtained data recorded by at least one mobile device of the subject over an observation period of multiple days, the passively obtained data comprising data regarding observed stride lengths of the subject; processing the passively obtained data to generate digital biomarker data; analyzing the digital biomarker data to determine whether the subject is experiencing cognitive decline; and generating a user notification to at least one of the subject and another user regarding the results of the analysis.
15. The computer-implemented method of aspect 14, wherein processing the passively obtained data comprises calculating a statistical skew of the observed stride lengths of the subject during the observation period, and wherein the digital biomarker data comprises the calculated statistical skew.
16. A computer-implemented method for detecting cognitive decline of a subject, the method comprising: receiving passively obtained data recorded by at least one mobile device of the subject over an observation period of multiple days, the passively obtained data comprising data regarding a number of exercise bouts during the observation period; analyzing the passively obtained data to determine whether the subject is experiencing cognitive decline; and generating a user notification to at least one of the subject and another user regarding the results of the analysis.
17. A computer-implemented method for detecting cognitive decline of a subject, the method comprising: receiving passively obtained data recorded by at least one mobile device of the subject over an observation period of multiple days, the passively obtained data comprising data regarding a number of times the subject viewed a mobile clock application for telling time on the at least one mobile device, wherein each time the subject viewed the mobile clock application is associated with a viewing duration; processing the passively obtained data to generate digital biomarker data; analyzing the passively obtained data to determine whether the subject is experiencing cognitive decline; and generating a user notification to at least one of the subject and another user regarding the results of the analysis.
18. The computer-implemented method of aspect 17, wherein processing the passively obtained data comprises calculating a viewing duration that is greater than or equal to a target percentage of the viewing durations associated with each time the subject viewed the mobile clock application during the observation period, and wherein the digital biomarker data comprises the calculated viewing duration.
19. The computer-implemented method of aspect 18, wherein the target percentage is between 90% and 100%.
20. The computer-implemented method of any of aspects 18-19, wherein the target percentage is between 93% and 97%.
21. The computer-implemented method of any of aspects 18-20, wherein the target percentage is 95%.
22. The computer-implemented method of any of aspects 17-21, wherein processing the passively obtained data comprises calculating a measure of statistical variability in the viewing durations associated with each time the subject viewed the mobile clock application during the observation period, and wherein the digital biomarker data comprises the calculated measure of statistical variability in the viewing durations.
23. The computer-implemented method of aspect 22, wherein the measure of statistical variability is an inter-quartile range.
24. The computer-implemented method of any of aspects 17-23, wherein processing the passively obtained data comprises summing all viewing durations associated with all the times the subject viewed the mobile clock application during the observation period to generate a total viewing duration, and wherein the digital biomarker data comprises the total viewing duration.
25. The computer-implemented method of any of aspects 17-24, wherein processing the passively obtained data comprises calculating, for each respective day in the observation period, a total daily viewing duration equal to the sum of all viewing durations associated with all the times the subject viewed the mobile clock application during the respective day, and calculating a measure of statistical variability in the calculated total daily viewing durations, and wherein the digital biomarker data comprises the calculated measure of statistical variability for the calculated total daily viewing durations.
26. The computer-implemented method of aspect 25, wherein the measure of statistical variability is an inter-quartile range.
27. A computer-implemented method for detecting cognitive decline of a subject, the method comprising: receiving passively obtained data recorded by at least one mobile device of the subject over an observation period of multiple days, the passively obtained data comprising data characterizing the manner in which the user types while composing outgoing messages sent by the communication device; processing the passively obtained data to generate digital biomarker data; analyzing the digital biomarker data to determine whether the subject is experiencing cognitive decline; and generating a user notification to at least one of the subject and another user regarding the results of the analysis.
28. The computer-implemented method of aspect 27, wherein processing the passively obtained data comprises calculating a typing speed excluding pauses, and wherein the digital biomarker data comprises the calculated typing speed.
29. The computer-implemented method of any of aspects 27-28, wherein processing the passively obtained data comprises calculating a mean number of words per sentence, and wherein the digital biomarker data comprises the calculated mean number of words.
30. A computer-implemented method for detecting cognitive decline of a subject, the method comprising: receiving passively-obtained time-series data of one or more user activities recorded by at least one mobile device of the subject over an observation period of multiple days; processing the passively obtained time-series data using a frequency analysis to convert the time-series data into a frequency power spectrum; calculating an amount of spectral energy in the frequency power spectrum between a first frequency threshold and a second frequency threshold; generating digital biomarker data based on the calculated amount of spectral energy; analyzing the digital biomarker data to determine whether the subject is experiencing cognitive decline; and generating a user notification to at least one of the subject and another user regarding the results of the analysis.
31. The computer-implemented method of aspect 30, wherein the first frequency threshold is less than 1/(24 hours) and the second frequency threshold is greater than 1/(24 hours).
32. The computer-implemented method of any of aspects 30-31, wherein the first frequency is greater than or equal to 1/(25 hours) and the second frequency threshold is less than or equal to 1/(23 hours).
33. The computer-implemented method of any of aspects 30-32, wherein the first frequency is greater than or equal to 1/(24 hours and 30 minutes) and the second frequency threshold is less than or equal to 1/(23 hours and 30 minutes).
34. The computer-implemented method of any of aspects 30-33, wherein the digital biomarker data comprises a ratio of (i) the calculated amount of spectral energy in the frequency power spectrum between the first frequency threshold and the second frequency threshold and (ii) the amount of spectral energy at all other frequencies in the frequency power spectrum.
35. The computer-implemented method of any of aspects 30-34, wherein the one or more user activities comprises at least one of phone calls, outgoing messages, incoming messages, mobile device unlocks, interaction with a mobile application, heart-rate, standing motions, steps, movement, movement while mobile device is unlocked, and movement while mobile device is locked.
36. The computer-implemented method of any of aspects 1-35, wherein the at least one mobile device of the subject comprises at least one of a smartwatch and a smartphone.
37. The computer-implemented method of any of aspects 1-36, wherein the cognitive decline is caused at least in part by Alzheimer's disease.
38. The computer-implemented method of any of aspects 1-37, wherein the analysis of the digital biomarker data is implemented using a convolutional neural network to determine whether the subject is experiencing cognitive decline.
39. The computer-implemented method of any of aspects 1-38, wherein the analysis of the digital biomarker data is implemented using one or more decision trees to determine whether the subject is experiencing cognitive decline.
40. The computer-implemented method of any of aspects 1-39, wherein the passively obtained data comprises at least a first category of data and a second category of data, wherein the first category of data is recorded at a first data collection frequency, and the second category of data is recorded at a second data collection frequency that is different from the first data collection frequency.
41. A processing device for detecting cognitive decline, the processing device comprising: one or more processors; and memory comprising instructions that, when executed, cause the one or more processors to perform the method of any of aspects 1-40.
42. A non-transitory computer-readable storage medium storing computer-executable instructions that, when executed by one or more processors, are configured to cause the one or more processors to perform the method of any of aspects 1-40.

## Claims

1. A processing device for detecting cognitive decline, the processing device comprising:
one or more processors; and
memory comprising instructions that, when executed, cause the one or more processors to perform a method comprising:
receiving passively obtained data recorded by at least one mobile device of the subject over an observation period of multiple days, the passively obtained data comprising data characterizing the manner in which the user types while composing outgoing messages sent by the communication device;
processing the passively obtained data to generate digital biomarker data;
analyzing the digital biomarker data to determine whether the subject is experiencing cognitive decline; and
generating a user notification to at least one of the subject and another user regarding the results of the analysis.

2. The processing device of claim 1, wherein processing the passively obtained data comprises calculating a typing speed excluding pauses, and wherein the digital biomarker data comprises the calculated typing speed.

3. The processing device of claim 1 or 2, wherein processing the passively obtained data comprises calculating a mean number of words per sentence, and wherein the digital biomarker data comprises the calculated mean number of words.

4. The processing device of any of claims 1-3, wherein the at least one mobile device of the subject comprises at least one of a smartwatch and a smartphone.

5. The processing device of any of claims 1-4, wherein the cognitive decline is caused at least in part by Alzheimer's disease.

6. The processing device of any of claims 1-5, wherein the analysis of the digital biomarker data is implemented using a convolutional neural network to determine whether the subject is experiencing cognitive decline.

7. The processing device of any of claims 1-6, wherein the analysis of the digital biomarker data is implemented using one or more decision trees to determine whether the subject is experiencing cognitive decline.

8. A non-transitory computer-readable storage medium storing computer-executable instructions that, when executed by one or more processors, are configured to cause the one or more processors to perform a method comprising:
receiving passively obtained data recorded by at least one mobile device of the subject over an observation period of multiple days, the passively obtained data comprising data characterizing the manner in which the user types while composing outgoing messages sent by the communication device;
processing the passively obtained data to generate digital biomarker data;
analyzing the digital biomarker data to determine whether the subject is experiencing cognitive decline; and
generating a user notification to at least one of the subject and another user regarding the results of the analysis.

9. The storage medium of claim 8, wherein processing the passively obtained data comprises calculating a typing speed excluding pauses, and wherein the digital biomarker data comprises the calculated typing speed.

10. The storage medium of claim 8 or 9, wherein processing the passively obtained data comprises calculating a mean number of words per sentence, and wherein the digital biomarker data comprises the calculated mean number of words.

11. The storage medium of any of claims 8-10, wherein the at least one mobile device of the subject comprises at least one of a smartwatch and a smartphone.

12. The storage medium of any of claims 8-11, wherein the cognitive decline is caused at least in part by Alzheimer's disease.

13. The storage medium of any of claims 8-12, wherein the analysis of the digital biomarker data is implemented using a convolutional neural network to determine whether the subject is experiencing cognitive decline.

14. The storage medium of any of claims 8-13, wherein the analysis of the digital biomarker data is implemented using one or more decision trees to determine whether the subject is experiencing cognitive decline.
